# EUROPEAN PATENT SPECIFICATION

(11) **EP 3 907 497 B1**
(45) Date of publication and mention of the grant of the patent: **02.08.2023**
(21) Application number: 21170123.0
(22) Date of filing: 23.04.2021
(51) Int. Cl.: G01N 21/64, G01J 3/28, A61B 5/00, G06T 7/90

(54) **APPARATUS AND METHOD FOR DISPLAYING AND/OR PRINTING IMAGES OF A SPECIMEN INCLUDING A FLUOROPHORE**
VORRICHTUNG UND VERFAHREN ZUM ANZEIGEN UND/ODER DRUCKEN VON BILDERN EINER PROBE MIT EINEM FLUOROPHOR
APPAREIL ET PROCÉDÉ D'AFFICHAGE ET/OU D'IMPRESSION D'IMAGES D'UN SPÉCIMEN COMPRENANT UN FLUOROPHORE

(30) Priority: 08.05.2020 DE 102020112572
(43) Date of publication of application: 10.11.2021
(73) Proprietor: Leica Microsystems CMS GmbH, 35578 Wetzlar (DE)
(72) Inventor: Pelzer, Patric, 35576 Wetzlar (DE)
(74) Representative: Schaumburg und Partner Patentanwälte mbB

(56) References cited:
- WO-A1-00/06774
- US-A- 5 127 730
- US-A- 5 817 462
- US-A1- 2002 102 617
- US-A1- 2014 267 672
- US-A1- 2017 237 958
- ADAMS S ET AL: "Fluorescence ratio imaging of cyclic AMP in single cells", NATURE, MACMILLAN JOURNALS LTD., ETC, LONDON, vol. 349, no. 6311, 21 February 1991 (1991-02-21), pages 694-697, XP002156501, ISSN: 0028-0836, DOI: 10.1038/349694A0
- MACVILLE MERRYN ET AL: "Spectral karyotyping, a 24-colour FISH technique for the identification of chromosomal rearrangements", HISTOCHEMISTRY AND CELL BIOLOGY, SPRINGER, BERLIN, DE, vol. 108, no. 4-5, 1 January 1997 (1997-01-01), pages 299-305, XP002408633, ISSN: 0948-6143, DOI: 10.1007/S004180050169
- LOEW L M: "CONFOCAL MICROSCOPY OF POTENTIOMETRIC FLUORESCENT DYES", CHEMICAL COMMUNICATIONS, CHEMICAL SOCIETY, LONDON, GB, vol. 38, 1 January 1993 (1993-01-01), pages 195-209, XP008056007, ISSN: 0009-241X

## Description

### Technical field

The invention relates to an apparatus for displaying and/or printing images of a specimen including a fluorophore. The invention further relates to a method for displaying and/or printing images of a specimen including a fluorophore.

### Background

Fluorescence microscopes comprise detection units configured to detect florescence light emitted by fluorophores introduced into a specimen in order to capture an image of the specimen. These detection units typically capture greyscale or monochromatic images. If more than one type of fluorophore is included within the specimen, optical filters are arranged in front of the detection unit in order to detect only the fluorescence light emitted by one of the fluorophores. Several images can be captures for different optical filter. Typically, one image for each type of fluorophore is acquired. However, since the detection unit only captures greyscale or monochromatic images. Even a trained observer will not always be able to infer which fluorophore was imaged based solely on the image content. As a means to ease the identification of the monochromatic images, these are often enhanced by false color-coding. False color-coding becomes even more important when the direct relation of different fluorophores needs to be compared by overlaying two or more images. In this case, only different false colors can be used as means for allowing to identify the fluorophore used.

US 2014/267672 A1 discloses in an imaging system for imaging a specimen. The imaging system comprises a micro-scope and an imaging apparatus configured for fluorescence imaging. A processing device of the imaging system is configured to generate a false color image in which different signals captured by the imaging apparatus are represented by a different color. The processing device is further configured to enhance the contrast of the false color image by maximizing color separation. In particular, the imaging apparatus is configured to enhance a contrast be-tween features in the false color image corresponding to different fluorophores.

Further reference is made to US 5 817 462 A which relates to coloring monochrome images captured by a fluorescence microscope for highlighting certain spectral wavelengths, and to US 2002/102617 A1 which relates to the use of false colors for coloring a monochrome image captured by a fluorescence microscope.

### Summary

It is an objective of the present invention to provide an apparatus and a method for displaying and/or printing images of a specimen including at least one fluorophore, that allows a user to distinguish and identify the features within the specimen, which are associated with the at least one fluorophore.

The aforementioned object is achieved by the subject-matter according to the independent claims. Advantageous embodiments are defined in the dependent claims and the following description.

In the present application hue is a property of a color. In particular, a color can be uniquely described by its hue, its brightness and its saturation.

The apparatus acquires the first and second images by capturing the fluorescence light emitted by the fluorophores. These images may be greyscale or monochrome images, since the detectors used to acquire images of fluorophores typically do not measure the wavelength of the emitted fluorescence light. The raw images show different features of the specimen, each feature being associated with either the first fluorophore or the second fluorophore. The apparatus then colors the first and second images in the first and second hue, respectively. This may be done by changing the hue of each pixel of the first and second raw images to the first and second hue, respectively, while retaining the brightness value. Alternatively, the brightness value may be changed as well. For example, the brightness value of each pixel of the first and second raw images may be adjusted according to a predetermined functional relationship or a predetermined lookup table. Thereby, the first and second false color images are obtained. The first and second false color images are monochromatic images in the first and second hues, respectively, and can now be distinguished by their respective hue. This allows a user to distinguish and identify the features within the specimen, which are associated with the first and second fluorophores.

Alternatively or additionally, lookup tables may be used to adjust the first and second hues according to the brightness value. This can be used to enhance contrast between different brightness values, e.g. by using a maximally discontinuous lookup table such as a Glasbey lookup table.

The sequence of the plurality of sequences is predetermined to maximize a contrast between the hues of the sequence. For example, the hues of the sequence may be equidistantly arranged on the color-wheel. This makes it easier for the user to distinguish the features within the specimen associated with the first and second fluorophores.

In another preferred embodiment the first hue is determined based on the spectral composition of the first fluorescence light and the second hue is determined based on the spectral composition of the second fluorescence light. For example, if the first fluorophore emits predominately blue fluorescence light and the second fluorophore emits predominately red fluorescence light, the first hue is determined to be a blue hue and the second hue is determined to be a red hue. In particular, the first and second hues are chosen according to the perceived hue of the first and second fluorescence light, respectively. Alternatively, the first and second hues are chosen according to a wavelength of the emissions maximum of the first and second fluorophores, respectively. In this embodiment, the user can easily relate the features of the specimen in the first and second false color images to the different fluorophores.

In another preferred embodiment at least one of the first and second hues is determined based on at least one optical filter used for acquiring at least one of the first or second raw images. For example, the first or second hue are chosen to correspond to a wavelength of the transmission maximum of the optical filter used for acquiring the first or second raw image, respectively. Preferably, the apparatus is configured to acquire the information required to determine the first and/or second hues, e.g. the transmission maximum of the optical filter, from the optical filter. For example, the optical filter might be provided with an identifier, e.g. bar code or an RFID-chip, identifying the optical filter. In this embodiment, no action by the user, such as a user input, is required in order to relate the features of the specimen in the first and second false color images with the different fluorophores. This greatly enhances user friendliness of the apparatus.

The sequence is predetermined such that hues of the sequence are suitable to be distinguished by a user with impaired color discrimination. The sequence is predetermined such, that is it possible for people with impaired color discrimination to unambiguously distinguish the colors of the sequence. This allows a user with impaired color discrimination to easily distinguish features within the specimen associated with the different fluorophores.

The apparatus comprises a user input unit configured to receive a user input. The sequence is selected from a plurality of sequences by the user input. For example, the user may choose a sequence with maximized contrast between the hues in order to distinguish the features of the specimen associated with different fluorophores.

The user can select the sequence according to their individual needs allowing for greater flexibility.

Alternatively, or additionally, the user input comprises at least one identifier for identifying at least one of the first and second fluorophores, and/or for identifying the spectral composition of at least one of the first and second fluorescence light. Thus, allowing the user to relate the features of the specimen with the different fluorophores.

In another preferred embodiment the image processing unit is configured to combine the first and second false color images into a single combined image. The output unit is configured to display and/or print the combined image. In this embodiment an image of the specimen is displayed or printed as the single combined image. Thus, the user can see the features of the specimen associated with the different fluorophores in relation to each other.

Preferably, the output unit is configured to display and/or print the combined image and the first and second false color images simultaneously. This allows for distinguishing features of the specimen associated with the different fluorophores even if they overlap in the combined image.

In another preferred embodiment the output unit is configured to display and/or print the combined image and the first and second raw images simultaneously. In particular, if the first and second raw images are greyscale images, the contrast of the first and second raw images will be higher than the contrast of the combined image. Thus, in this embodiment, the user can distinguish even faint features of the specimen associated with the different fluorophores.

In another preferred embodiment the image processing unit is configured to generate a first color-marked image by adding a first color marker to the first raw image, in particular by arranging a first colored frame around the first raw image. The first color marker having the first hue. The image processing unit is further configured to generate a second color-marked image by adding a second color marker to the second raw image, in particular by arranging a second colored frame around the second raw image. The second color marker frame having the second hue. The output unit is configured to display and/or print the first and second color-marked images. The color markings make it easy to relate the first and second raw images to their associated fluorophore without coloring them. Thereby, the high contrast of the raw images is preserved while still allowing to distinguish the features of the specimen associated with the different fluorophores.

Optionally, the image acquisition unit is configured to receive the first and second raw images from a fluorescence microscope, in particular by means of a digital telecommunications network. This network might be the internet or a local area network. This embodiment allows the apparatus to be separate from fluorescence microscope enhancing the ease of use.

Another aspect not covered by the claimed invention relates to an apparatus for displaying and/or printing images of a specimen including a fluorophore. The apparatus comprising: An image acquisition unit, configured to acquire a raw image captured by detecting fluorescence light emitted by the fluorophore, wherein the raw image comprises a plurality of pixel, each pixel having a brightness value. An image processing unit, configured to determine a hue based on the spectral composition of the fluorescence light, and to generate a false color image by assigning a single hue to each pixel of the raw image. An output unit, configured to display and/or print the false color image.

The hue is determined based on the spectral composition of the fluorescence light. This means, for example, if the fluorophore emits predominately blue fluorescence light, the hue is determined to be a blue hue. If on the other hand the fluorophore emits predominately red fluorescence light, the hue is determined to be a red hue. In particular, the hue is chosen according to the perceived hue of the fluorescence light. Alternatively, the hue is chosen according to a wavelength of the emissions maximum of the fluorophore. Thus, the user can easily relate the features of the specimen in the false color image to the specific type of fluorophore used.

In a preferred embodiment not covered by the claimed invention the image processing unit is configured to determine the hue based on an optical filter used for acquiring the raw image. Preferably, the apparatus is configured to acquire the information required to determine the hue, e.g. the transmission maximum of the optical filter, from the optical filter. For example, the optical filter might be provided with an identifier, e.g. bar code or an RFID-chip, identifying the optical filter. In this embodiment, no action by the user, such as a user input, is required in order to determine the optical filter used. This greatly enhances user friendliness of the apparatus.

The invention further relates to a method for displaying and/or printing an image of a specimen including a first fluorophore and a second fluorophore as set out in the appended set of claims.

The method has the same advantages as the apparatus' claimed and can be supplemented using the features of the dependent claims directed at the apparatus'.

### Short Description of the Figures

Hereinafter, specific embodiments are described referring to the drawings, wherein:
Figure 1 is a schematic diagram of an apparatus for displaying images of a specimen and a fluorescence microscope;
Figure 2 is a schematic diagram of raw images received by the apparatus, and false color images and the combined image generated by the apparatus according to Figure 1; and
Figure 3 a schematic diagram of the raw images received by the apparatus and first and second color-marked images generated by the apparatus according to Figure 1.

### Detailed description

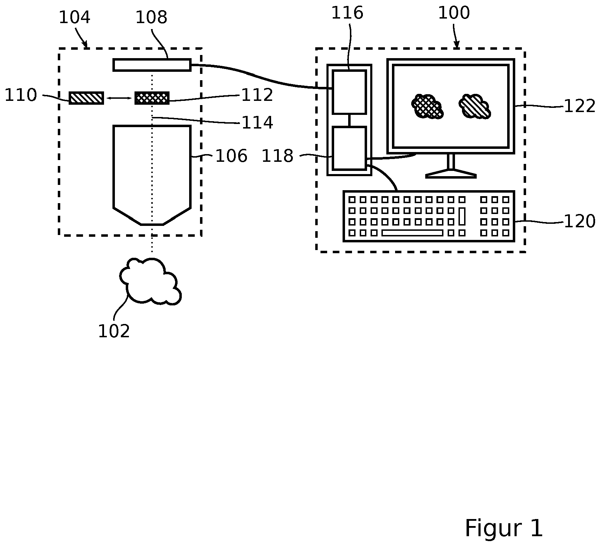

Figure 1 shows a schematic diagram of an apparatus 100 for displaying images of a specimen 102 including a first fluorophore and a second fluorophore according to an embodiment. Figure 1 further shows a fluorescence microscope 104 configured to capture images of the specimen 102.

The fluorescence microscope 104 comprises an optical system 106 configured to image the specimen 102 onto a detection unit 108. The fluorescence microscope 104 further comprises a set of exchangeable optical filters 110, 112 that can be brought into a light path 114 of the fluorescence microscope 104. A first optical filter 110 of the exchangeable optical filters 110, 112 is only transparent to first fluorescence light emitted by the first fluorophore. A second optical filter 112 of the exchangeable optical filters 110, 112 is only transparent to second fluorescence light emitted by the second fluorophore. Alternatively, the fluorescence microscope 104 may comprise other means for selecting specific wavelengths, e.g. a dispersion element such as a grating.

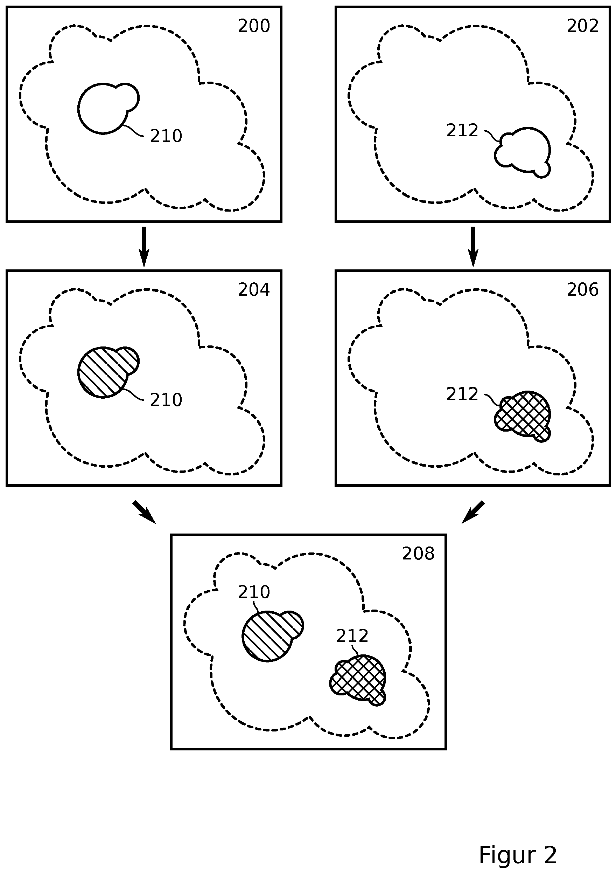

By introducing the first optical filter 110 into the light path 114 of the fluorescence microscope 104, only features of the specimen 102 including the first fluorophore are imaged by the detection unit 108. Accordingly, by introducing the second optical filter 112 into the light path 114 of the fluorescence microscope 104, only features of the specimen 102 including the second fluorophore are imaged. The features of the specimen 102 comprising the first fluorophore are henceforth called first features 210 (see Figure 2) and the features of the specimen 102 comprising the second fluorophore are henceforth called second features 212 (see Figure 2). The images of the first and second features 210, 212 captured by the detection unit 108 are called first and second raw images 200, 202 (see Figure 2), respectively.

Alternatively, the first and second features 210, 212 are imaged by first exciting only the first fluorophore, e.g. with a laser having a first wavelength, and capturing the first raw image 200, and then exciting only the second fluorophore, e.g. with a laser having a second wavelength, and capturing the second raw image 202. In this embodiment, the set of exchangeable filters 110, 112 is not needed.

The apparatus 100 comprises an image acquisition unit 116, an image processing unit 118, a user input unit 120, and an output unit 122. The image acquisition unit 116 is configured to receive the first and second raw images 200, 202 from the fluorescence microscope 104. The image processing unit 118 is configured to generate first and second false color images 204, 206 (see Figure 2) from the first and second raw images 200, 202, respectively. In order to generate the first and second false color images 204, 206 the image processing unit 118 first selects a first hue and a second hue from a predetermined sequence of hues, the first and second hues being different from each other.

The first and second hues are determined based on the first and second optical filters 110, 112, respectively. This means, if e.g. the first optical filter 110 is transmissive to predominately blue light, the first hue is selected to be a blue hue. The first and second hues may also be selected based on the first and second fluorophores, respectively. For example, the specific type of the first fluorophore and the second fluorophore may be entered into the apparatus 100 by a user via the user input unit 120. The first and second hues are then selected based on the user input. The first and second hues are determined based on the spectral composition of the first and second fluorescence light, respectively.

Alternatively the first and second hues are determined independent from the spectral composition of the first and second fluorescence light. Alternatively the first and second hues are selected from a sequence that is predetermined to maximize a contrast between the hues of the sequence. Alternatively the first and second hues are selected from a sequence that is predetermined such that hues of the sequence are suitable to be distinguished by a user with impaired color discrimination. The selection of both the sequence and the first and second hues may be determined by a user input received via the user input unit 120.

The image processing unit 118 is configured to generate the first false color image 204 by assigning the first hue to each pixel of the first raw image 200, and to generate the second false color image 206 by assigning the second hue to each pixel of the second raw image 202. The brightness value of each pixel is kept. That is, the brightness value of each pixel in the first and second false color images 204, 206 is the same as the brightness level of the corresponding pixel in the first and second raw images 200, 202, respectively. Alternatively, the brightness level of each pixel may be adjusted according to a functional dependency or a lookup table. The image processing unit 118 also is configured to combine the first and second false color images 204, 206 into a single combined image 208 (see Figure 2). The generation of the first and second false color images 204, 206 and the combined image 208 is shown in Figure 2.

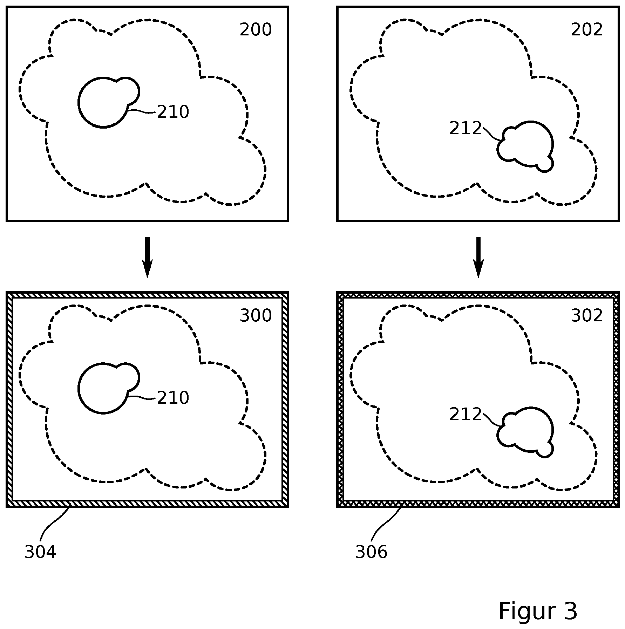

The image processing is further configured to generate to generate a first and second color-marked images 300, 302 (see Figure 3). The first color-marked image 300 is generated by adding a first color marker 304 (see Figure 3) having the first hue to the first raw image 200. The second color-marked image 302 is generated by adding a second color marker 306 (see Figure 3) having the second hue to the second raw image 202. The first and second color markers 304, 306 are colored frames. The generation of the first and second color-marked images 300, 302 is shown in Figure 3.

The output unit 122 is exemplary designed as a computer monitor and configured to display at least one of the first and second raw images 200, 202, the first and second false color images 204, 206, the first and second color-marked images 300, 302 and the combined image 208 depending on the user input received via the user input unit 120. The output unit 122 is further configured to display any combination of the aforementioned images depending on the user input received via the user input unit 120.

Figure 2 shows a schematic diagram of the first and second raw images 200, 202 received by the apparatus 100. Figure 2 furthers shows a schematic diagram of the first and second false color images 204, 206 and the combined image 208 generated by the apparatus 100. An outline of the specimen 102 is shown as a dashed line in Figure 2.

The first raw image 200 comprises the first features 210, that is a feature of the specimen 102, e.g. a cell of cluster of cells, including the first fluorophore. The second raw image 202 comprises the second features 212, that is a feature of the specimen 102 different from the first features 210 including the second fluorophore. The first false color image 204 comprises the first features 210 colored in the first hue. The first hue is shown in Figure 2 a hatch pattern. The second false color image 206 comprises the second features 212 colored in the second hue. The second hue is shown in Figure 2 a crosshatch pattern. The combined image 208 comprises both the first and second features 210, 212.

Figure 3 shows a schematic diagram of the first and second raw images 200, 202 received by the apparatus 100 and the first and second color-marked images 300, 302 generated by the apparatus 100.

The first color-marked image 300 comprises the first features 210 and the first color marker 304. The first color marker 304 is exemplary formed as a colored frame having the first hue. The second color-marked image 302 comprises the second features 212 and the second color marker 306. The second color marker 306 is exemplary formed as a colored frame having the second hue. Alternatively, the first and second color markers 304, 306 may take any other suitable form, e.g. a colored circle having the first or second hue, respectively, arranged in a corner of the respective color-marked image.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100: Apparatus
- 102: Specimen
- 104: Fluorescence microscope
- 106: Optical system
- 108: Detection unit
- 110, 112: Optical filter
- 114: Light path
- 116: Image acquisition unit
- 118: Image processing unit
- 120: Unser input unit
- 122: Output unit
- 200, 202: Raw image
- 204, 206: False color image
- 208: Combined image
- 210,212: Feature
- 300, 302: Color-marked image
- 304, 306: Color marker

## Claims

1. An apparatus (100) for displaying and/or printing images of a specimen (102) including a first fluorophore and a second fluorophore, comprising:
an image acquisition unit (116) configured to acquire a first raw image (200) captured by detecting first fluorescence light emitted by the first fluorophore and a second raw image (202) captured by detecting second fluorescence light emitted by the second fluorophore, said second fluorescence light having a spectral composition being different from a spectral composition of the first fluorescence light, wherein each of the first and second raw images (200, 202) comprises a plurality of pixels each pixel having a brightness value;
an image processing unit (118) configured
to select a first hue and a second hue from a predetermined sequence of hues,
to generate a first false color image (204) by assigning the first hue to each pixel of the first raw image (200), and
to generate a second false color image (206) by assigning the second hue to each pixel of the second raw image (202),
wherein the first and second hues are different from each other;
an output unit (122) configured to display and/or print the first and second false color images (204, 206); and
a user input unit (120) configured to receive a user input, wherein the sequence is selected from a plurality of sequences by the user input.

2. The apparatus (100) according to claim 1, wherein the sequence is predetermined to maximize a contrast between the hues of the sequence.

3. The apparatus (100) according to claim 1 or 2, wherein the first hue is determined based on the spectral composition of the first fluorescence light and the second hue is determined based on the spectral composition of the second fluorescence light.

4. The apparatus (100) according to one of the preceding claims, wherein at least one of the first and second hues is determined based on at least one optical filter used for acquiring at least one of the first or second raw images (200, 202).

5. The apparatus (100) according to claim 1 or 2, wherein the sequence is predetermined such that hues of the sequence are suitable to be distinguished by a user with impaired color discrimination.

6. The apparatus (100) according to one of the preceding claims, wherein the user input comprises at least one identifier for identifying at least one of the first and second fluorophores, and/or for identifying the spectral composition of at least one of the first and second fluorescence light.

7. The apparatus (100) according to one of the preceding claims, wherein the image processing unit (118) is configured to combine the first and second false color images (204, 206) into a single combined image (208); and
wherein the output unit (122) is configured to display and/or print the combined image (208).

8. The apparatus (100) according to claim 7, wherein the output unit (122) is configured to display and/or print the combined image (208) and the first and second false color images (204, 206) simultaneously.

9. The apparatus (100) according to claim 7 or 8, wherein the output unit (122) is configured to display and/or print the combined image (208) and the first and second raw images (200, 202) simultaneously.

10. The apparatus (100) according to one of the preceding claims, wherein the image processing unit (118) is configured
to generate a first color-marked image (300) by adding a first color marker (304) to the first raw image (200), in particular by arranging a first colored frame around the first raw image (200), said first color marker (304) having the first hue, and
to generate a second color-marked image (302) by adding a second color marker (306) to the second raw image (202), in particular by arranging a second colored frame around the second raw image (202), said second color marker (306) frame having the second hue; and
wherein the output unit (122) is configured to display and/or print the first and second color-marked images (300, 302).

11. The apparatus (100) according to one of the preceding claims, wherein the image acquisition unit (116) is configured to receive the first and second raw images (200, 202) from a fluorescence microscope (104), in particular by means of a digital telecommunications network.

12. A method for displaying and/or printing an image of a specimen (102) including a first fluorophore and a second fluorophore, comprising:
acquiring a first raw image (200) captured by detecting first fluorescence light emitted by the first fluorophore and a second raw image (202) captured by detecting second fluorescence light emitted by the second fluorophore, said second fluorescence light having a spectral composition being different from a spectral composition of the first fluorescence light, wherein the first and second raw images (200, 202) each comprise a plurality of pixels each pixel having a brightness value;
selecting a predetermined sequence of hues from a plurality of sequences by a user input;
selecting a first hue and a second hue from the sequence of hues;
generating a first false color image (204) by assigning the first hue to each pixel of the first raw image (200);
generating a second false color image (206) by assigning the second hue to each pixel of the second raw image (202), wherein the first and second hues are different from each other; and
displaying and/or printing the first and second false color images (204, 206).

## Patentansprüche

1. Einrichtung (100) zum Anzeigen und/oder Drucken von Bildern einer Probe (102), die ein erstes Fluorophor und ein zweites Fluorophor beinhaltet, umfassend:
eine Bilderfassungseinheit (116), die ausgelegt ist zum Erfassen eines ersten Rohbildes (200), das durch Detektieren eines ersten Fluoreszenzlichts aufgenommen wird, das durch das erste Fluorophor emittiert wird, und eines zweiten Rohbildes (202), das durch Detektieren eines zweiten Fluoreszenzlichts aufgenommen wird, das durch das zweite Fluorophor emittiert wird, wobei das zweite Fluoreszenzlicht eine spektrale Zusammensetzung aufweist, die sich von einer spektralen Zusammensetzung des ersten Fluoreszenzlichts unterscheidet, wobei sowohl das erste als auch das zweite Rohbild (200, 202) eine Vielzahl von Pixeln umfasst, wobei jedes Pixel einen Helligkeitswert aufweist;
eine Bildverarbeitungseinheit (118), die ausgelegt ist zum
Auswählen eines ersten Farbtons und eines zweiten Farbtons aus einer vorbestimmten Sequenz von Farbtönen,
Erzeugen eines ersten Falschfarbenbildes (204) durch Zuweisen des ersten Farbtons zu jedem Pixel des ersten Rohbildes (200), und
Erzeugen eines zweiten Falschfarbenbildes (206) durch Zuweisen des zweiten Farbtons zu jedem Pixel des zweiten Rohbildes (202),
wobei sich der erste und der zweite Farbton voneinander unterscheiden;
eine Ausgabeeinheit (122), die ausgelegt ist zum Anzeigen und/oder Drucken des ersten und des zweiten Falschfarbenbildes (204, 206); und
eine Benutzereingabeeinheit (120), die ausgelegt ist zum Empfangen einer Benutzereingabe, wobei die Sequenz aus einer Vielzahl von Sequenzen durch die Benutzereingabe ausgewählt wird.

2. Einrichtung (100) nach Anspruch 1, wobei die Sequenz vorbestimmt ist, um einen Kontrast zwischen den Farbtönen der Sequenz zu maximieren.

3. Einrichtung (100) nach Anspruch 1 oder 2, wobei der erste Farbton basierend auf der spektralen Zusammensetzung des ersten Fluoreszenzlichts bestimmt wird und der zweite Farbton basierend auf der spektralen Zusammensetzung des zweiten Fluoreszenzlichts bestimmt wird.

4. Einrichtung (100) nach einem der vorstehenden Ansprüche, wobei mindestens einer des ersten und zweiten Farbtons basierend auf mindestens einem optischen Filter bestimmt wird, das zum Erfassen mindestens eines des ersten oder zweiten Rohbildes (200, 202) verwendet wird.

5. Einrichtung (100) nach Anspruch 1 oder 2, wobei die Sequenz vorbestimmt ist, sodass Farbtöne der Sequenz für die Unterscheidung durch einen Benutzer mit eingeschränkter Farbdiskriminierung geeignet sind.

6. Einrichtung (100) nach einem der vorstehenden Ansprüche, wobei die Benutzereingabe mindestens eine Kennung zum Identifizieren mindestens eines des ersten und zweiten Fluorophors und/oder zum Identifizieren der spektralen Zusammensetzung mindestens eines des ersten und zweiten Fluoreszenzlichts umfasst.

7. Einrichtung (100) nach einem der vorstehenden Ansprüche, wobei die Bildverarbeitungseinheit (118) ausgelegt ist zum Kombinieren des ersten und zweiten Falschfarbenbildes (204, 206) in ein einzelnes kombiniertes Bild (208); und
wobei die Ausgabeeinheit (122) ausgelegt ist zum Anzeigen und/oder Drucken des kombinierten Bildes (208).

8. Einrichtung (100) nach Anspruch 7, wobei die Ausgabeeinheit (122) ausgelegt ist zum gleichzeitigen Anzeigen und/oder Drucken des kombinierten Bildes (208) und des ersten und zweiten Falschfarbenbildes (204, 206).

9. Einrichtung (100) nach Anspruch 7 oder 8, wobei die Ausgabeeinheit (122) ausgelegt ist zum gleichzeitigen Anzeigen und/oder Drucken des kombinierten Bildes (208) und des ersten und zweiten Rohbildes (200, 202).

10. Einrichtung (100) nach einem der vorstehenden Ansprüche, wobei die Bildverarbeitungseinheit (118) ausgelegt ist zum
Erzeugen eines ersten farblich markierten Bildes (300) durch Hinzufügen einer ersten Farbmarkierung (304) zu dem ersten Rohbild (200), insbesondere durch Anordnen eines ersten farbigen Rahmens um das erste Rohbild (200), wobei die erste Farbmarkierung (304) den ersten Farbton aufweist, und
Erzeugen eines zweiten farblich markierten Bildes (302) durch Hinzufügen einer zweiten Farbmarkierung (306) zu dem zweiten Rohbild (202), insbesondere durch Anordnen eines zweiten farbigen Rahmens um das zweite Rohbild (202), wobei der Rahmen der zweiten Farbmarkierung (306) den zweiten Farbton aufweist; und
wobei die Ausgabeeinheit (122) ausgelegt ist zum Anzeigen und/oder Drucken des ersten und zweiten farblich markierten Bildes (300, 302).

11. Einrichtung (100) nach einem der vorstehenden Ansprüche, wobei die Bilderfassungseinheit (116) ausgelegt ist zum Empfangen des ersten und zweiten Rohbildes (200, 202) von einem Fluoreszenzmikroskop (104), insbesondere mittels eines digitalen Telekommunikationsnetzwerks.

12. Verfahren zum Anzeigen und/oder Drucken eines Bildes einer Probe (102), die ein erstes Fluorophor und ein zweites Fluorophor beinhaltet, umfassend:
Erfassen eines ersten Rohbildes (200) , das durch Detektieren eines ersten Fluoreszenzlichts aufgenommen wird, das durch das erste Fluorophor emittiert wird, und eines zweiten Rohbildes (202), das durch Detektieren eines zweiten Fluoreszenzlichts aufgenommen wird, das durch das zweite Fluorophor emittiert wird, wobei das zweite Fluoreszenzlicht eine spektrale Zusammensetzung aufweist, die sich von einer spektralen Zusammensetzung des ersten Fluoreszenzlichts unterscheidet, wobei sowohl das erste als auch das zweite Rohbild (200, 202) eine Vielzahl von Pixeln umfasst, wobei jedes Pixel einen Helligkeitswert aufweist;
Auswählen einer vorbestimmten Sequenz von Farbtönen aus einer Vielzahl von Sequenzen durch eine Benutzereingabe; Auswählen eines ersten Farbtons und eines zweiten Farbtons aus der Sequenz von Farbtönen;
Erzeugen eines ersten Falschfarbenbildes (204) durch Zuweisen des ersten Farbtons zu jedem Pixel des ersten Rohbildes (200);
Erzeugen eines zweiten Falschfarbenbildes (206) durch Zuweisen des zweiten Farbtons zu jedem Pixel des zweiten Rohbildes (202), wobei sich der erste und der zweite Farbton voneinander unterscheiden; und
Anzeigen und/oder Drucken des ersten und des zweiten Falschfarbenbildes (204, 206).

## Revendications

1. Appareil (100) d'affichage et/ou d'impression d'images d'un échantillon (102) comportant un premier fluorophore et un second fluorophore, comprenant :
une unité d'acquisition d'images (116) configurée pour acquérir une première image brute (200) capturée en détectant une première lumière de fluorescence émise par le premier fluorophore et une seconde image brute (202) capturée en détectant une seconde lumière de fluorescence émise par le second fluorophore, ladite seconde lumière de fluorescence ayant une composition spectrale différente d'une composition spectrale de la première lumière de fluorescence, dans lequel chacune des première et seconde images brutes (200, 202) comprend une pluralité de pixels, chaque pixel ayant une valeur de luminosité ;
une unité de traitement d'images (118) configurée pour
sélectionner une première teinte et une seconde teinte à partir d'une séquence prédéterminée de teintes,
générer une première image fausse couleur (204) en attribuant la première teinte à chaque pixel de la première image brute (200), et
générer une seconde image fausse couleur (206) en attribuant la seconde teinte à chaque pixel de la seconde image brute (202),
dans lequel les première et seconde teintes sont différentes l'une de l'autre ;
une unité de sortie (122) configurée pour afficher et/ou imprimer les première et seconde images fausse couleur (204, 206) ; et
une unité d'entrée d'utilisateur (120) configurée pour recevoir une entrée d'utilisateur, dans lequel la séquence est sélectionnée parmi une pluralité de séquences par l'entrée d'utilisateur.

2. Appareil (100) selon la revendication 1, dans lequel la séquence est prédéterminée pour maximiser un contraste entre les teintes de la séquence.

3. Appareil (100) selon la revendication 1 ou 2, dans lequel la première teinte est déterminée en fonction de la composition spectrale de la première lumière de fluorescence et la seconde teinte est déterminée en fonction de la composition spectrale de la seconde lumière de fluorescence.

4. Appareil (100) selon l'une des revendications précédentes, dans lequel au moins une des première et seconde teintes est déterminée en fonction au moins d'un filtre optique utilisé pour acquérir au moins une des première ou seconde images brutes (200, 202).

5. Appareil (100) selon la revendication 1 ou 2, dans lequel la séquence est prédéterminée de telle sorte que des teintes de la séquence puissent être distinguées par un utilisateur présentant une déficience de discrimination des couleurs.

6. Appareil (100) selon l'une des revendications précédentes, dans lequel l'entrée d'utilisateur comprend au moins un identifiant pour identifier au moins un des premier et second fluorophores et/ou identifier la composition spectrale d'au moins une des première et seconde lumières de fluorescence.

7. Appareil (100) selon l'une des revendications précédentes, dans lequel l'unité de traitement d'images (118) est configurée pour combiner les première et seconde images fausse couleur (204, 206) en une seule image combinée (208) ; et
dans lequel l'unité de sortie (122) est configurée pour afficher et/ou imprimer l'image combinée (208).

8. Appareil (100) selon la revendication 7, dans lequel l'unité de sortie (122) est configurée pour afficher et/ou imprimer l'image combinée (208) et les première et seconde images fausse couleur (204, 206) simultanément.

9. Appareil (100) selon la revendication 7 ou 8, dans lequel l'unité de sortie (122) est configurée pour afficher et/ou imprimer simultanément l'image combinée (208) et les première et seconde images brutes (200, 202) .

10. Appareil (100) selon l'une des revendications précédentes, dans lequel l'unité de traitement d'images (118) est configurée pour
générer une première image colorée (300) en ajoutant un premier marqueur de couleur (304) à la première image brute (200), notamment en disposant un premier cadre coloré autour de la première image brute (200), ladite première image couleur (304) ayant la première teinte, et
générer une seconde image colorée (302) en ajoutant un second marqueur de couleur (306) à la seconde image brute (202), notamment en disposant un second cadre coloré autour de la seconde image brute (202), ladite seconde image couleur (306) ayant la seconde teinte ; et
dans lequel l'unité de sortie (122) est configurée pour afficher et/ou imprimer les première et seconde images colorées (300, 302).

11. Appareil (100) selon l'une des revendications précédentes, dans lequel l'unité d'acquisition d'images (116) est configurée pour recevoir les première et seconde images brutes (200, 202) à partir d'un microscope à fluorescence (104), notamment au moyen d'un réseau de télécommunications numériques.

12. Procédé d'affichage et/ou d'impression d'une image d'un échantillon (1()2) comportant un premier fluorophore et un second fluorophore, comprenant :
l'acquisition d'une première image brute (200) capturée en détectant une première lumière de fluorescence émise par le premier fluorophore et une seconde image brute (202) capturée en détectant une seconde lumière de fluorescence émise par le second fluorophore, ladite seconde lumière de fluorescence ayant une composition spectrale différente d'une composition spectrale de la première lumière de fluorescence, dans lequel les première et seconde images brutes (200, 202) comprennent chacune une pluralité de pixels, chaque pixel ayant une valeur de luminosité ;
la sélection d'une séquence prédéterminée de teintes parmi une pluralité de séquences par une entrée d'utilisateur ;
la sélection d'une première teinte et d'une seconde teinte dans la séquence de teintes ;
la génération d'une première image fausse couleur (204) en attribuant la première teinte à chaque pixel de la première image brute (200) ;
la génération d'une seconde image fausse couleur(206) en attribuant la seconde teinte à chaque pixel de la seconde image brute (202), dans lequel les première et seconde teintes sont différentes l'une de l'autre ; et
l'affichage et/ou l'impression des première et seconde images fausse couleur (204, 206).
